# FASCICULE DE BREVET EUROPEEN

(11) **EP 1 331 924 B1**
(45) Date de publication et mention de la délivrance du brevet: **13.08.2008**
(21) Numéro de dépôt: 01982552.0
(22) Date de dépôt: 25.10.2001
(51) Int. Cl.: A61K 8/31, A61K 8/67, A61Q 19/08

(54) **UTILISATION DE BETA-CAROTENE ET DE LYCOPENE POUR TRAITER LES SIGNES CUTANES DU VIEILLISSEMENT VIA L'INHIBITION DE L'EXPRESSION DE LA METALLOPROTEINASE DE TYPE 1 DE LA MATRICE EXTRACELLULAIRE**
VERWENDUNG VON BETA-CAROTIN UND LYCOPIN ZUR BEHANDLUNG DES HAUTALTERNS DURCH INHIBIERUNG DER AUSWIRKUNG DER METALLOPROTEINASE DES TYPS 1 DER EXTRAZELLULAREN MATRIX
USE OF BETA-CAROTENE AND LYCOPENE FOR TREATING AGEING SYMPTOMS VIA THE INHIBITION OF THE EXPRESSION OF THE METALLOPROTEINASE OF TYPE 1 OF THE EXTRACELLULAR MATRIX

(30) Priorité: 26.10.2000 FR 0013753
(43) Date de publication de la demande: 06.08.2003
(73) Titulaire: L'ORÉAL, 75008 Paris (FR); Nestec S.A., 1800 Vevey (CH)
(72) Inventeur: BRETON, Lionel, F-78000 Versailles (FR); BAUR, Markus, CH-1603 Aran (CH)
(74) Mandataire: Le Coupanec, Pascale A.M.P.
(86) Numéro de dépôt international: PCT/FR2001/003315
(87) Numéro de publication internationale: WO 2002/034232

(56) Documents cités:
- EP-A- 0 712 630
- EP-A- 0 981 969
- WO-A-01/26668
- WO-A-97/47278
- WO-A-99/48386
- DE-A- 19 942 774
- GB-A- 1 323 800
- US-A- 5 895 652
- US-A- 5 976 568
- US-A- 6 048 846
- US-A- 6 130 254

## Description

L'invention se rapporte à l'utilisation de l'association de β-carotène et de lycopene dans une composition ou pour la préparation d'une composition, l'association étant destinée à traiter, les signes cutanés du vieillissement, en particulier la dégradation de la peau et/ou des muqueuses via l'inhibition de l'expression de la metalloproteinase de type 1 de la matrice extracellulaire.

Chez les mammifères en général, particulièrement chez l'homme, la peau est constituée de deux compartiments à savoir un compartiment en relation avec l'extérieur, l'épiderme, et un compartiment profond qui sert de soutien à l'épiderme, le derme.

L'épiderme naturel est composé principalement de trois types de cellules qui sont les kératinocytes, très majoritaires, les mélanocytes et les cellules de Langerhans. Chacun dé ces types cellulaires contribue par ses fonctions propres au rôle essentiel joué dans l'organisme par la peau.

Le derme fournit à l'épiderme un support solide. C'est également son élément nourricier. Il est principalement constitué de fibroblastes et d'une matrice extracellulaire composée elle-même principalement de collagène, d'élastine et d'une substance, dite substance fondamentale, composants synthétisés par le fibroblaste. On y trouve aussi des leucocytes, des mastocytes ou encore des macrophages tissulaires. II est également traversé par des vaisseaux sanguins et des fibres nerveuses. Dans une peau normale, c'est à dire non pathologique ni cicatricielle, le fibroblaste est à l'état quiescent, c'est à dire non prolifératif, peu actif d'un point de vue métabolique et non mobile.

Les fibres de collagène assurent en grande partie la solidité du derme. Ces fibres sont constituées de fibrilles scellées les unes aux autres, formant ainsi plus de dix types de structures différentes. La solidité du derme est en grande partie due à l'enchevêtrement des fibres de collagène tassées les unes contre les autres en tous sens. Les fibres de collagène participent également à l'élasticité et surtout à la tonicité de la peau et/ou des muqueuses.
Les fibres de collagène sont constamment renouvelées mais ce renouvellement diminue avec l'âge ce qui entraîne un amincissement du derme. Cet amincissement du derme est également dû à des causes pathologiques comme par exemple l'hypersécrétion d'hormones corticoïdes, certaines pathologies ou encore des carences vitaminiques. Il est également admis que des facteurs extrinsèques comme les rayons ultraviolets, le tabac ou certains traitements (Glucocorticoïdes, vitamine D et dérivés par exemple) ont également un effet sur la peau et sur son taux de collagène.

Cependant, divers facteurs entraînent la dégradation du collagène, avec toutes les conséquences que l'on peut envisager sur la structure et/ou la fermeté de la peau et/ou des muqueuses.

Bien que très résistantes, les fibres de collagène sont sensibles à certaines enzymes appelées collagénases. Une dégradation des fibres de collagène entraîne l'apparence de peau molle et ridée que l'on cherche depuis toujours à combattre, particulièrement chez l'être humain, préférant l'apparence d'une peau lisse et tendue.

Les collagénases font partie d'une famille d'enzymes appelées métalloprotéinases (MMPs) qui sont elles-mêmes les membres d'une famille d'enzymes protéolytiques (endoprotéases) qui possèdent un atome de zinc coordonné à 3 résidus cystéine et une méthionine dans leur site actif et qui dégradent les composants macromoléculaires de la matrice extracellulaire et des lames basales à pH neutre (collagène, élastine, etc. ...). Très largement répandues dans le monde vivant, ces enzymes sont présentes, mais faiblement exprimées, dans des situations physiologiques normales comme la croissance des organes et le renouvellement des tissus.

Leur surexpression et leur activation sont cependant liées à de nombreux processus, parfois pathologiques, qui impliquent la destruction et le remodelage de la matrice. Cela entraîne soit une résorption non contrôlée de la matrice extracellulaire, soit inversement l'installation d'un état de fibrose.

La famille des métalloprotéinases est constituée de plusieurs groupes bien définis basés sur leurs ressemblances en terme de structure et de spécificité de substrat (voir Woessner J. F., Faseb Journal, vol. 5, 1991, 2145). Parmi ces groupes, on peut citer les collagénases destinées à dégrader les collagènes fibrillaires (MMP-1 ou collagénase interstitielle, MMP-8 ou collagénase de neutrophile, MMP-13 ou collagénase 3), les gélatinases qui dégradent le collagène de type IV ou toute forme de collagène dénaturé (MMP-2 ou gélatinase A (72 kDa), MMP-9 ou gélatinase B (92 kDa)), les stromélysines (MMP-3) dont le large spectre d'activité s'adresse aux protéines de la matrice extracellulaire telles que les glycoprotéines (fibronectine, laminine), les protéoglycannes, etc., ou encore les métalloprotéinases membranaires.
L'exposition prolongée aux rayonnements ultraviolets, particulièrement aux rayonnements ultraviolets de type A et/ou B, a pour effet une stimulation de l'expression des collagénases, particulièrement de la MMP-1. C'est là une des composantes du vieillissement cutané photo-induit.

Par ailleurs à la ménopause, les principales modifications concernant le derme sont une diminution du taux de collagène et de l'épaisseur dermique. Cela entraîne chez la femme ménopausée un amincissement de la peau et/ou des muqueuses. La femme ressent alors une sensation de "peau sèche" ou de peau qui tire et l'on constate une accentuation des rides et ridules de surface. La peau présente un aspect rugueux à la palpation. Enfin la peau présente une souplesse diminuée.

On comprend alors à la lecture de ce qui précède l'importance du collagène dans la structure des tissus, particulièrement de la peau et/ou des muqueuses, et l'importance qu'il y a à combattre sa dégradation pour ainsi lutter contre les signes cutanés du vieillissement, qu'il soit chronologique ou photo-induit, et ses conséquences, comme par exemple l'amincissement du derme et/ou la dégradation des fibres de collagène ce qui entraînent l'apparence de peau molle, distendue et ridée.

Par signes cutanés du vieillissement on entend toutes modifications de l'aspect extérieur de la peau dues au vieillissement qu'il soit chronobiologique et/ou photo-induit, comme par exemple les rides et ridules, la peau flétrie, la peau molle, la peau amincie, la peau terne et sans éclat, le manque d'élasticité et/ou de tonus de la peau, mais également toutes modifications internes de la peau qui ne se traduisent pas systématiquement par un aspect extérieur modifié, comme par exemple toutes dégradations internes de la peau, particulièrement des fibres de collagène, consécutives à une exposition aux rayonnements ultra-violets, qui peuvent avoir comme conséquence l'amincissement du derme.

Un des buts de la présente invention est donc de pouvoir disposer d'un produit qui permette de traiter, chez les mammifères en général, particulièrement chez l'homme, les signes cutanés du vieillissement qu'il soit chronobiologique ou photo-induit, particulièrement l'amincissement du derme et/ou la dégradation des fibres de collagène, par un effet stimulateur de la synthèse du collagène via un effet inhibiteur des collagénases et si possible pas d'effets secondaires notables.

De manière surprenante, la Demanderesse a maintenant découvert que l'association de β-carotène et de lycopène permet de traiter les signes cutanés du vieillissement qu'il soit chronobiologique ou photo-induit, particulièrement l'amincissement du derme ét/ou la dégradation des fibres de collagène, par un effet inhibiteur de la métalloprotéinase de type 1 de la matrice extracellulaire.

Une propriété remarquable de l'association de l'invention est qu'elle présente des effets dans des proportions plus importantes que celles raisonnablement attendues de la simple addition des effets de chacun de ces composants pris séparément.

Un avantage de cette propriété est de permettre une utilisation dans la composition de l'invention d'une quantité de chacun des produits inférieure à ce qu'il est généralement admis d'utiliser.

A la connaissance de la demanderesse l'activité inhibitrice sur la métalloprotéinase de type 1 de la matrice extracellulaire par cette association n'a jamais été décrite.

Ainsi, l'invention a pour premier objet l'utilisation selon la revendication 1.

L'invention a pour second objet l'utilisation selon la revendication 1 dans laquelle l'association est destinée à lutter contre les dégradations du collagène.

L'invention a également pour objet l'utilisation selon la revendication 1 dans laquelle l'association est destinée à traiter les atteintes cutanées liées au vieillissement, notamment aux atteintes cutanées de la ménopause.

L'invention a également pour objet l'utilisation selon la revendication 1 dans laquelle l'association est destinée à lutter contre les rides et ridules.

L'invention a également pour objet l'utilisation selon la revendication 1 dans laquelle, l'association est destinée à lutter contre la peau flétrie.

L'invention a également pour objet l'utilisation selon la revendication 1 dans laquelle l'association est destinée à lutter contre la peau molle.

L'invention a également pour objet l'utilisation selon la revendication 1 dans laquelle l'association est destinée à lutter contre la peau amincie.

L'invention a également pour objet l'utilisation selon la revendication 1 dans laquelle, l'association est destinée à lutter contre la peau terne et sans éclat.

L'invention a également pour objet l'utilisation selon la revendication 1 dans laquelle l'association est destinée à lutter contre le manque d'élasticité et/ou de tonus de la peau.

Quelle que soit l'utilisation envisagée de l'invention, celle-ci peut être de manière préventive à l'atteinte qu'elle vise à traiter.

Il est bien évident que l'invention s'adresse aux mammifères en général et particulièrement aux êtres humains.

Le lycopène est un pigment naturel que l'on trouve dans les fruits mûrs, particulièrement dans la tomate. Il appartient à la famille des caroténoïdes et sa structure est proche de celle du β-carotène.
Le rôle du lycopène dans la maturation des fruits est connu dans l'art antérieur.

Le lycopène est utilisé dans des compositions à activité bronzante pour son rôle sur la synthèse de mélanine (WO 97/47278), dans des compositions destinées au traitement de la chevelure et/ou de l'acné pour son activité sur les 5α-réductases (JP-2940964) ou encore comme agent anti-radicalaire (JP-A-8-283136.
Le lycopène peut être sous forme chimique cis ou trans.

Les autres caroténoïdes utilisés selon l'invention, avec ou sans activité provitaminique A, peuvent être d'origine naturelle ou synthétique.
Par origine naturelle, on entend le caroténoïde, à l'état pur ou en solution quelle qu'en soit sa concentration dans ladite solution, obtenu à partir d'un élément naturel.
Selon un mode préférentiel de l'invention, on utilise un extrait riche en Lycopène, comme par exemple un extrait de tomate.
Par origine synthétique, on entend le caroténoïde, à l'état pur ou en solution quelle qu'en soit sa concentration dans ladite solution, obtenu par synthèse chimique.

Lorsque le caroténoïde est d'origine naturelle, il peut être obtenu à partir de matériel végétal issu de plante entière cultivée *in vivo* ou issu de culture *in vitro.*

Par culture *in vivo* on entend toute culture de type classique c'est à dire en sol à l'air libre ou en serre, ou encore hors sol.
Par culture *in vitro,* on entend l'ensemble des techniques connues de l'homme du métier qui permet de manière artificielle l'obtention d'un végétal ou d'une partie d'un végétal. La pression de sélection imposée par les conditions physico-chimiques lors de la croissance des cellules végétales *in vitro* permet d'obtenir un matériel végétal standardisé et disponible tout au long de l'année contrairement aux plantes cultivées *in vivo,*

Préférentiellement selon l'invention, on utilise un végétal issu de culture *in vivo.*

Toute méthode d'extraction connue de l'homme du métier peut être utilisée pour préparer le caroténoïde utilisé selon l'invention.

Le caroténoïde peut être en solution alcoolique, notamment éthanolique.

Le caroténoïde peut également être en solution lipidique (huile) ou lipoalcoolique.

A titre d'exemple, selon l'invention on utilise un caroténoïde sans activité provitaminique A sous la forme d'un extrait de tomate riche en lycopène, préparé par la société Métaphar, commercialisé sous la dénomination LycOMato® constitué d'un extrait d'oléorésine (phase grasse) contenant 6% de lycopène pur.

On peut également utiliser selon l'invention toute préparation contenant du lycopène ayant pour objectif d'améliorer la biodisponibilité de ce dernier et/ou tous nouveaux procédés de fabrication et/ou formulation et/ou d'encapsulation du Lycopène.

La quantité de β-carotène utilisable selon l'invention est bien entendu fonction de l'effet recherché et peut donc varier dans une large mesure.

Pour donner un ordre de grandeur, dans la composition selon l'invention le β-carotène à l'état pur est en une quantité représentant de 10⁻¹²% à 20% du poids total de la composition et préférentiellement en une quantité représentant de 10⁻¹⁰% à 10% du poids total de la composition.

La quantité de lycopène utilisable selon l'invention est bien entendu fonction de l'effet recherché et peut donc varier dans une large mesure.
Pour donner un. ordre de grandeur, dans la composition selon l'invention le lycopène à l'état pur est en une quantité représentant de 10⁻¹²% à 20% du poids total de la composition et préférentiellement en une quantité représentant de 10⁻¹⁰% à 10% du poids total de la composition.

Bien entendu l'homme du métier, s'il utilise le β-carotène et/ou le lycopène sous la forme d'une solution, un extrait végétal par exemple, sait ajuster la quantité de solution qu'il utilise dans sa composition afin que la quantité finale de caroténoïde dans la composition soit en accord avec les quantités utilisables précédemment définies.

La composition de l'invention peut être sous toutes formes galéniques imaginables, adaptées aussi bien à une application topique sur la peau et/ou les muqueuses et/ou les cheveux qu'à une administration par la voie orale.
De manière préférentielle, la composition de l'invention est destinée à une administration par la voie orale.

La composition de l'invention peut être une composition cosmétique ou dermatologique. Préférentiellement selon l'invention, la composition est une composition cosmétique. La composition est une composition cosmétique car elle est destinée à améliorer l'aspect cutané général de l'individu qui en fait usage.
Très préférentiellement la composition de l'invention est une composition cosmétique destinée à une administration par la voie orale.

Pour une administration par la voie orale, la composition de l'invention peut se présenter sous toutes les formes adaptées, particulièrement sous forme d'une solution buvable, d'un sirop, d'un comprimé, d'une dragée, d'une gélule ou encore d'une capsule ou encore un aliment nutritionnel ou d'un complément nutritionnel.
Ladite composition peut comprendre en outre au moins un excipient approprié adapté à l'administration orale.

Pour une administration par application topique sur la peau, les cheveux et/ou les poils et/ou les muqueuses, la composition selon l'invention comprend bien évidemment un support cosmétiquement acceptable, c'est à dire un support compatible avec la peau, les muqueuses, les ongles, les cheveux et peut se présenter sous toutes les formes galéniques normalement utilisées pour une application topique, notamment sous forme d'une solution aqueuse, hydroalcoolique ou huileuse, d'une émulsion huile-dans-eau ou eau-dans-huile
ou multiple, d'un gel aqueux ou huileux, d'un produit anhydre liquide, pâteux ou solide, d'une dispersion d'huile dans une phase aqueuse à l'aide de sphérules, ces sphérules pouvant être des nanoparticules polymériques telles que les nanosphères et les nanocapsules ou mieux des vésicules lipidiques de type ionique et/ou non-ionique.

Cette composition peut être plus ou moins fluide et avoir l'aspect d'une crème blanche ou colorée, d'une pommade, d'un lait, d'une lotion, d'un sérum, d'une pâte, d'une mousse. Elle peut éventuellement être appliquée sur la peau sous forme d'aérosol. Elle peut également se présenter sous forme solide, et par exemple sous forme de stick. Elle peut être utilisée comme produit de soin, comme produit de nettoyage, comme produit de maquillage ou encore comme simple produit déodorant.

De façon connue, la composition de l'invention peut contenir les adjuvants habituels dans les domaines cosmétiques et dermatologique, tels que les gélifiants hydrophiles ou lipophiles, les actifs hydrophiles ou lipophiles, les conservateurs, les antioxydants, les solvants, les parfums, les charges, les filtres, les pigments, les agents chélateurs, les absorbeurs d'odeur et les matières colorantes. Les quantités de ces différents adjuvants sont celles classiquement utilisées dans les domaines considérés, et par exemple de 0,01 % à 20 % du poids total de la composition. Ces adjuvants, selon leur nature, peuvent être introduits dans la phase grasse, dans la phase aqueuse, dans les vésicules lipidiques et/ou dans les nanoparticules.

Lorsque la composition de l'invention est une émulsion, la proportion de la phase grasse peut aller de 5 % à 80 % en poids, et de préférence de 5 % à 50 % du poids total de la composition. Les huiles, les émulsionnants et les coémulsionnants utilisés dans la composition sous forme d'émulsion sont choisis parmi ceux classiquement utilisés dans le domaine considéré. L'émulsionnant et le coémulsionnant sont présents, dans la composition, en une proportion allant de 0,3 % à 30 % en poids, et de préférence de 0,5 % à 20 % du poids total de la composition.

Comme huiles utilisables dans l'invention, on peut citer les huiles minérales, les huiles d'origine végétale (huile d'abricot, huile de tournesol), les huiles d'origine animale, les huiles de synthèse, les huiles siliconées et les huiles fluorées (perfluoropolyéthers). On peut aussi utiliser comme matières grasses des alcools gras (alcool cétylique), des acides gras, des cires (cire d'abeilles).

Comme émulsionnants et coémulsionnants utilisables dans l'invention, on peut citer par exemple les esters d'acide gras et de polyéthylène glycol tels que le stéarate de PEG-40, le stéarate de PEG-100, les esters d'acide gras et de polyol tels que le stéarate de glycéryle et le tristéarate de sorbitane.

Comme gélifiants hydrophiles, on peut citer en particulier les polymères carboxyvinyliques (carbomer), les copolymères acryliques tels que les copolymères d'acrylates/alkylacrylates, les polyacrylamides, les polysaccharides, les gommes naturelles et les argiles, et, comme gélifiants lipophiles, on peut citer les argiles modifiées comme les bentones, les sels métalliques d'acides gras, la silice hydrophobe et les polyéthylènes.

La composition peut contenir d'autres actifs hydrophiles comme les protéines ou les hydrolysats de protéine, les acides aminés, les polyols, l'urée, l'allantoïne, les sucres et les dérivés de sucre, les extraits végétaux et les hydroxy-acides.

Comme actifs lipophiles, on peut utiliser le rétinol (vitamine A) et ses dérivés, le tocophérol (vitamine E) et ses dérivés, les acides gras essentiels, les céramides, les huiles essentielles, l'acide salicylique et ses dérivés ou encore les vitamines B1, B6 et /ou B12.

Il est également possible d'utiliser dans la composition de l'invention la vitamine C (ou acide ascorbique) et ses dérivés (esters, sels, etc.).

On peut également y ajouter un extrait végétal riche en isoflavonoïdes, comme par exemple l'extrait de soja (Glycina max) disponible auprès de Archer Daniels Midland Company sous la dénomination Novasoy®.

Une composition préférée comprend entres autres ingrédients un extrait végétal riche en isoflavonoïdes, de la vitamine C et de l'α-tocophérol.

Encore plus préférentiellement, la composition comprend entre autres ingrédients l'association de β-carotène et de lycopène, de l'extrait de soja (Novasoy® de la société Archer Daniels Midland Company), de la vitamine C et de l'acétate d'α-tocophérol.

II est également possible d'utiliser en outre dans la composition des composés choisis parmi
- les oligo-éléments ;
- les hormones végétales ;
- les agents antagonistes de calcium, comme le vérapamil et le Diltiazem ;
- des capteurs de radicaux OH, tels que le dirnéthylsulfoxyde ;
- des ouvreurs de canaux chlore ;
- des extraits de végétaux tels que ceux d'Iridacées, de Rosacées ou de Rosmarinus ;
- des extraits de micro-organismes dont en particulier des extraits bactériens comme ceux de bactéries filamenteuses non photosynthétiques ;

A la liste ci-dessus, d'autres composés peuvent également être rajoutés, à savoir par exemple les ouvreurs de canaux potassiques tels que le diazoxyde et le minoxidil, la spiroxazone, des phospholipides comme la lécithine, les acides linoléique et linolénique, l'acide salicylique et ses dérivés décrits dans le brevet français FR 2 581 542, comme les dérivés de l'acide salicylique porteurs d'un groupement alcanoyle ayant de 2 à 12 atomes de carbone en position 5 du cycle benzénique, des acides hydroxycarboxyliques ou cétocarboxyliques et leurs esters, des lactones et leurs sels correspondants, l'anthraline, des caroténoïdes, les acides eicosatétraénoïque et eicosatriénoïque ou leurs esters et amides, la vitamine D et ses dérivés.

On peut, entre autres, ajouter à la composition d'autres agents actifs destinés notamment à la prévention et/ou au traitement des affections cutanées. Parmi ces agents actifs, on peut citer à titre d'exemple :
- les agents modulant la différenciation et/ou la prolifération et/ou la pigmentation cutanée tels que l'acide rétinoïque et ses isomères, le rétinol et ses esters, la vitamine D et ses dérivés, les oestrogènes tels que l'oestradiol, l'acide kojique ou l'hydroquinone ;
- les antibactériens tels que le phosphate de clindamycine, l'érythromycine ou les antibiotiques de la classe des tétracyclines ;
- les agents modulant l'adhésion bactérienne sur la peau et /ou les muqueuses tels que le miel, notamment le miel d'acacias et certains dérivés de sucres ;
- les antiparasitaires, en particulier le métronidazole, le crotamiton ou les pyréthrinoïdes ;
- les antifongiques, en particulier les composés appartenant à la classe des imidazoles tels que l'éconazole, le kétoconazole ou le miconazole ou leurs sels, les composés polyènes, tels que l'amphotéricine B, les composés de la famille des allylamines, tels que la terbinafine, ou encore l'octopirox ;
- les agents antiviraux tels que l'acyclovir ;
- les agents anti-inflammatoires stéroïdiens, tels que l'hydrocortisone, le valérate de bétaméthasone ou le propionate de clobétasol, ou les agents anti-inflammatoires non-stéroïdiens tels que l'ibuprofène et ses sels, le diclofénac et ses sels, l'acide acétylsalicylique, l'acétaminophène ou l'acide glycyrrhétinique ;
- les agents anesthésiques tels que le chlorhydrate de lidocaïne et ses dérivés ;
- les agents antiprurigineux comme la thénaldine, la triméprazine ou la cyproheptadine ;
- les agents kératolytiques tels que les acides alpha- et bêta-hydroxycarboxyliques ou bêta-cétocarboxyliques, leurs sels, amides ou esters et plus particulièrement les hydroxy-acides tels que l'acide glycolique, l'acide lactique, l'acide salicylique, l'acide citrique et de manière générale les acides de fruits, et l'acide n-octanoyl-5-salicylique ;
- les agents anti-radicaux libres, tels que les superoxyde dismutases, certains chélatants de métaux ou l'acide ascorbique et ses esters ;
- les anti-séborrhéiques tels que la progestérone ;
- les antipelliculaires comme l'octopirox ou la pyrithione de zinc ;
- les antiacnéiques comme l'acide rétinoïque ou le peroxyde de benzoyle.
- des substances telles que les antagonistes de substance P, de CGRP ou de bradykinine ou les inhibiteurs de NO synthase ou encore les inhibiteurs de canaux sodiques, composés décrits comme étant actifs dans le traitement des peaux sensibles et comme présentant des effets anti-irritants, en particulier vis-à-vis de composés irritants éventuellement présents dans les compositions.

Comme actifs, on peut utiliser notamment les hydratants tels que les polyols (par exemple la glycérine), les vitamines (par exemple le D-panthénol), les agents anti-inflammatoires, les agents apaisants (allantoïne, eau de bleuet), les filtres UVA et UVB, les agents matifiants (par exemple les polydiméthylorganosiloxanes partiellement réticulés vendus sous le nom KSG® par Shin Etsu) et leurs mélanges.

On peut aussi ajouter des actifs antirides, et notamment des produits tenseurs tels que les protéines végétales et leurs hydrolysats, en particulier l'extrait de protéines de soja vendu sous le nom d'Eleseryl® par la société LSN ou le dérivé d'avoine vendu sous la dénomination Reductine® par la société Silab.

La peau étant constituée de bien d'autres composants que le collagène et les fibroblastes, il s'avère intéressant, lorsque l'on utilise l'association de l'invention, de favoriser en même temps la synthèse de ces autres composants comme par exemple les lipides et/ou de favoriser la prolifération d'autres composantes cellulaires comme par exemple les kératinocytes.

Une composition résultant de l'utilisation selon l'invention peut être destinée à une utilisation dans le domaine cosmétique ou dermatologique, préférentiellement dans le domaine cosmétique.

On peut citer comme produit stimulant la synthèse des lipides les hormones végétales, comme les auxines, ou des composés d'origine végétale, comme l'acide cinnamique et comme produit stimulant la prolifération des kératinocytes des composés d'origine végétale, comme le phloroglucinol.

Ainsi, les compositions peuvent comprendre en plus de l'association de l'invention, de l'acide cinnamique ou ses dérivés et/ou une hormone végétale, particulièrement une auxine choisie parmi l'acide indolacétique (IAA), l'acide 4-chloroindole-3-acétique (4-CI-IAA), l'acide phénylacétique (PAA), l'acide indole-3-butyrique (IBA), l'acide 2,4-dichlorophenoxyacétique (2,4-D), l'acide α-naphtalèneacétique (α-NAA), l'acide β-naphtoxyacétique, l'indole éthanol, l'idole acétaldéhyde et l'indole acétonitrile et/ou un composé végétal comme le phloroglucinol.

L'invention a aussi pour objet l'utilisation de l'association telle que définie en revendication 1 et d'au moins un autre produit stimulant la synthèse des lipides et/ou la prolifération des kératinocytes dans une composition ou pour la préparation d'une composition; l'association étant destinée à inhiber l'expression de la métalloprotéinase de type 1 à et en particulier à traiter, de manière préventive et/ou curative, les signes cutanés du vieillissement, à lutter contre les dégradations du collagène, à traiter les atteintes cutanées liées à la ménopause, à lutter contre les rides et ridules, à lutter contre la peau flétrie, à lutter contre la peau molle, à lutter contre la peau amincie, à lutter contre la peau terne et/ou sans éclat, à lutter contre le manque d'élasticité et/ou de tonus de la peau,

Les exemples et compositions suivants illustrent l'invention sans la limiter aucunement. Dans les compositions les proportions indiquées sont des pourcentages en poids.

**Composition 1 - Capsules molles :**

| | |
|---|---|
| Excipients : | |
| Huile de Soja | 40 mg |
| Huile de Germe de Blé | 85 mg |
| Lécithines de Soja | 25 mg |
| Vitamines: | |
| Tocophérols naturels | 3 mg |
| Vitamine C | 50 mg |
| Composants : | |
| Lycopène | 6 mg . |
| Béta-carotène | 10 mg |

**Composition 2 : Crème de soin (émulsion huile dans eau)**

| | |
|---|---|
| Lycopène à 6% (Lycomato®) | 0,001 % |
| Béta-carotène | 0,01 % |
| Stéarate de glycérol | 2,00 % |
| Polysorbate 60 (Tween 60^{®} vendu par la société ICI) | 1,00 % |
| Acide stéarique | 1,40 % |
| Triéthanolamine | 0,70 % |
| Carbomer | 0,40 % |
| Fraction liquide du beurre de karité | 12,00 % |
| Perhydrosqualène | 12,00 % |
| Antioxydant | 0,05 % |
| Parfum | 0,50 % |
| Conservateur | 0,30 % |
| Eau | qsp 100,00 % |

**Composition 3 : Gel pour le soin du visage**

| | |
|---|---|
| Lycopène à 6 % (Lycomato®) | 0,001 % |
| Béta-carotène | 0,01 % |
| Hydroxypropylcellulose (Klucel H^{®} vendu par la société Hercules) | 1,00 % |
| Antioxydant | 0,05 % |
| Isopropanol | 40,00 % |
| Conservateur | 0,30 % |
| Eau | qsp 100,00 % |
| Composition 4 : Crème de soin (émulsion huile-dans-eau) | |
| Lycopène à 6 % (Lycomato®) | 0,001 % |
| Béta-carotène | 0,01 % |
| Stéarate de glycérol | 2,00 % |
| Polysorbate 60 (Tween 60^{®} vendu par la société ICI) | 1,00 % |
| Acide stéarique | 1,40 % |
| Acide glycyrrhétinique | 2,00 % |
| Triéthanolamine | 0,70% |
| Carbomer | 0,40 % |
| Fraction liquide du beurre de karité | 12,00 % |
| Huile de tournesol | 10,00 % |
| Antioxydant | 0,05 % |
| Parfum | 0,50 % |
| Conservateur | 0,30 % |
| Eau | qsp 100,00 % |

## Revendications

1. Utilisation d'au moins une association de β-carotène et de lycopène dans une composition ou pour la préparation d'une composition, l'association étant destinée à inhiber l'expression de la métalloprotéinase de type 1 de la matrice extracellulaire.

2. Utilisation selon la revendications précédentes dans laquelle la composition est destinée à lutter contre les dégradations du collagène.

3. Utilisation selon l'une au moins des revendications précédentes dans laquelle la composition est destinée à traiter les signes cutanés du vieillissement.

4. Utilisation selon l'une au moins des revendications précédentes dans laquelle la composition est destinée à traiter les atteintes cutanées liées à la ménopause.

5. Utilisation selon l'une au moins des revendications précédentes dans laquelle la composition est destinée à lutter contre les rides et ridules.

6. Utilisation selon l'une au moins des revendications précédentes dans laquelle la composition est destinée à lutter contre la peau flétrie.

7. Utilisation selon l'une au moins des revendications précédentes dans laquelle la composition est destinée à lutter contre la peau molle.

8. Utilisation selon l'une au moins des revendications précédentes dans laquelle la composition est destinée à lutter contre la peau amincie.

9. Utilisation selon l'une au moins des revendications précédentes dans laquelle la composition est destinée à lutter contre la peau terne et sans éclats.

10. Utilisation selon l'une au moins des revendications précédentes dans laquelle la composition est destinée à lutter contre le manque d'élasticité et/ou de tonus de la peau.

11. Utilisation selon l'une quelconque des revendications précédentes, **caractérisée par le fait que** le β-carotène à l'état pur est en une quantité représentant de 10⁻¹²% à 20 % en poids du poids total de la composition.

12. Utilisation selon l'une quelconque des revendications précédentes, **caractérisée par le fait que** le β-carotène à l'état pur est en une quantité représentant de 10⁻¹⁰ % à 10 % en poids du poids total de la composition.

13. Utilisation selon l'une quelconque des revendications précédentes, **caractérisée par le fait que** le lycopène à l'état pur est en une quantité représentant de 10⁻¹² % à 20 % du poids total de la composition.

14. Utilisation selon l'une quelconque des revendications précédentes, **caractérisée par le fait que** le lycopène à l'état pur est en une quantité représentant de 10⁻¹⁰ % à 10 % du poids total de la composition.

15. Utilisation selon l'une quelconque des revendications précédentes, **caractérisée par le fait que** la composition est destinée à une administration par la voie orale.

16. Utilisation selon l'une quelconque des revendications précédentes, **caractérisée par le fait que** la composition se présente sous la forme d'une solution buvable, d'un sirop, d'un comprimé, d'une dragée, d'une gélule, d'une capsule ou encore un aliment nutritionnel ou d'un complément nutritionnel.

## Claims

1. Use of at least one combination of β-carotene and lycopene in a composition or for the preparation of a composition, the combination being for inhibiting the expression of extracellular matrix metalloproteinase type 1.

2. Use according to the preceding claim, in which the composition is for combating collagen breakdown.

3. Use according to at least one of the preceding claims, in which the composition is for treating signs of ageing of the skin.

4. Use according to at least one of the preceding claims, in which the composition is for treating cutaneous conditions related to the menopause.

5. Use according to at least one of the preceding claims, in which the composition is for combating wrinkles and fine lines.

6. Use according to at least one of the preceding claims, in which the composition is for combating wizened skin.

7. Use according to at least one of the preceding claims, in which the composition is for combating flaccid skin.

8. Use according to at least one of the preceding claims, in which the composition is for combating thinned skin.

9. Use according to at least one of the preceding claims, in which the composition is for combating dull, lifeless skin.

10. Use according to at least one of the preceding claims, in which the composition is for combating lack of elasticity and/or tonicity of the skin.

11. Use according to any one of the preceding claims, **characterized in that** the β-carotene in the pure state is in an amount representing from 10⁻¹²% to 20% by weight of the total weight of the composition.

12. Use according to any one of the preceding claims, **characterized in that** the β-carotene in the pure state is in an amount representing from 10⁻¹⁰% to 10% by weight of the total weight of the composition.

13. Use according to any one of the preceding claims, **characterized in that** the lycopene in the pure state is in an amount representing from 10⁻¹²% to 20% by weight of the total weight of the composition.

14. Use according to any one of the preceding claims, **characterized in that** the lycopene in the pure state is in an amount representing from 10⁻¹⁰% to 10% by weight of the total weight of the composition.

15. Use according to any one of the preceding claims, **characterized in that** the composition is for oral administration.

16. Use according to any one of the preceding claims, **characterized in that** the composition is in the form of an oral solution, a syrup, a tablet, a dragée, a gel capsule, a capsule or else a nutritional food or a nutritional supplement.

## Patentansprüche

1. Verwendung von mindestens einer Kombination von β-Carotin und Lycopin in einer Zusammensetzung oder zur Herstellung einer Zusammensetzung, wobei die Kombination dazu bestimmt ist, die Expression der Metalloproteinase vom Typ 1 der extrazellulären Matrix zu hemmen.

2. Verwendung nach dem vorhergehenden Anspruch, wobei die Zusammensetzung dazu bestimmt ist, die Zersetzungen des Collagens zu bekämpfen.

3. Verwendung nach mindestens einem der vorhergehenden Ansprüche, wobei die Zusammensetzung dazu bestimmt ist, die Anzeichen der Hautalterung zu bekämpfen.

4. Verwendung nach mindestens einem der vorhergehenden Ansprüche, wobei die Zusammensetzung dazu bestimmt ist, die mit der Menopause zusammenhängenden Hautschäden zu bekämpfen.

5. Verwendung nach mindestens einem der vorhergehenden Ansprüche, wobei die Zusammensetzung dazu bestimmt ist, die Falten und Fältchen zu bekämpfen.

6. Verwendung nach mindestens einem der vorhergehenden Ansprüche, wobei die Zusammensetzung dazu bestimmt ist, die welke Haut zu bekämpfen.

7. Verwendung nach mindestens einem der vorhergehenden Ansprüche, wobei die Zusammensetzung dazu bestimmt ist, die schlaffe Haut zu bekämpfen.

8. Verwendung nach mindestens einem der vorhergehenden Ansprüche, wobei die Zusammensetzung dazu bestimmt ist, die dünner gewordenen Haut zu bekämpfen.

9. Verwendung nach mindestens einem der vorhergehenden Ansprüche, wobei die Zusammensetzung dazu bestimmt ist, die matte und glanzlose Haut zu bekämpfen.

10. Verwendung nach mindestens einem der vorhergehenden Ansprüche, wobei die Zusammensetzung dazu bestimmt ist, die mangelnde Elastizität und/oder den mangelnden Tonus der Haut zu bekämpfen,

11. Verwendung nach mindestens einem der vorhergehenden Ansprüche, **dadurch gekennzeichnet, dass** das β-Carotin in reinem Zustand in einer Menge vorhanden ist, die 10⁻¹² Gew.-% bis 20 Gew.-% des Gesamtgewichts der Zusammensetzung darstellt.

12. Verwendung nach mindestens einem der vorhergehenden Ansprüche, **dadurch gekennzeichnet, dass** das β-Carotin in reinem Zustand in einer Menge vorhanden ist, die 10⁻¹⁰ Gew.-% bis 10 Gew.-% des Gesamtgewichts der Zusammensetzung darstellt.

13. Verwendung nach mindestens einem der vorhergehenden Ansprüche, **dadurch gekennzeichnet, dass** das Lycopin in reinem Zustand in einer Menge vorhanden ist, die 10⁻¹² Gew.-% bis 20 Gew.-% des Gesamtgewichts der Zusammensetzung darstellt.

14. Verwendung nach mindestens einem der vorhergehenden Ansprüche, **dadurch gekennzeichnet, dass** das Lycopin in reinem Zustand in einer Menge vorhanden äst, die 10⁻¹⁰ Gew.-% bis 10 Gew.-% des Gesamtgewichts der Zusammensetzung darstellt.

15. Verwendung nach mindestens einem der vorhergehenden Ansprüche, **dadurch gekennzeichnet, dass** die Zusammensetzung zur oralen Verabreichung bestimmt ist.

16. Verwendung nach mindestens einem der vorhergehenden Ansprüche, **dadurch gekennzeichnet, dass** die Zusammensetzung in Form einer trinkbaren Lösung, eines Sirups, einer Tablette, eines Dragees, einer Gelatinekapsel, einer Kapsel oder auch eines Nahrungsmittel oder Nahrungsergänzungsmittels vorliegt.
